(19) Europäisches Patentamt
European Patent Office
Office européen des brevets

(11) EP 0 197 766 B1

(12) EUROPEAN PATENT SPECIFICATION

(45) Date of publication and mention
of the grant of the patent:
24.07.1996 Bulletin 1996/30

(51) Int Cl.6: C07D 303/14, C07D 301/19

(21) Application number: 86302466.7

(22) Date of filing: 03.04.1986

(54) **Catalytic asymmetric epoxidation**

Asymetrische katalytische Epoxidierung

Epoxydation catalytique asymétrique

(84) Designated Contracting States:
AT BE CH DE FR GB IT LI LU NL SE

(30) Priority: 04.04.1985 US 719776

(43) Date of publication of application:
15.10.1986 Bulletin 1986/42

(73) Proprietor: MASSACHUSETTS INSTITUTE OF
TECHNOLOGY
Cambridge, MA 02139 (US)

(72) Inventors:
• Hanson, Robert M.
Cambridge Massachusetts 02139 (US)
• Ko, Soo Young
Cambridge Massachusetts 02140 (US)
• Sharpless, Karl B.
Brookline Massachusetts 02146 (US)

(74) Representative: Ford, Michael Frederick et al
MEWBURN ELLIS
York House
23 Kingsway
London WC2B 6HP (GB)

(56) References cited:
EP-A- 0 046 033

• JOURNAL OF ORGANIC CHEMISTRY, vol. 48,
no. 20, 7th October 1983, pages 3607,3608,
American Chemical Society, Easton, P.A., US;
J.G. HILL et al.: "Anhydrous tert-butyl
hydroperoxide in toluene: the preferred reagent
for applications requiring dry TBHP"
• CHEMISCHE BERICHTE, vol. 113, no. 11, 3rd
November 1980, pages 3662-3665, Verlag
Chemie GmbH, Weinheim, DE; H. LANGHALS et
al.: "Die Trocknung von tert-Butylhydroperoxid
nach einem einfachen, erstmals gefahrlosen
Verfahren"

## Description

The synthetic production of a wide variety of drugs and naturally occurring products is dependent upon the introduction of an oxygen functionality at a particular site. Frequently, the oxygen functionality may be associated with a second functionality in the vicinal position. For many compounds of interest, one or both of these sites may be chiral. The epoxide functionality is a particularly attractive functionality for providing the two substituents. Furthermore, where the epoxide can be provided as a particular enantiomer or diastereomer, by appropriate selection of reagents and conditions, one can provide for the correct stereoisomers at the two positions. There is, therefore, substantial interest in ways to prepare epoxides in high yield providing for asymmetric formation of the epoxide.

US-A-4,471,130 (EP-A-0 046 033) describes asymmetric epoxidation of ethylenic alcohols employing, for the most part, stoichiometric amounts of metal alkoxide catalyst. Numerous other articles have been published employing the process described in the above-cited patent, using a wide variety of olefinic alcohols for epoxide formation, substantially following the stoichiometric conditions described in the cited patent.

As in EP-A-0 046 033, in the present invention allylic alcohols are asymmetrically epoxidized by a method employing an organic hydroperoxide, a titanium dural glycol alkoxide catalyst, where the glycol is optically active, and the allylic alcohol, in an inert organic solvent. The invention is characterized in that the reaction is carried out in the presence of activated molecular sieves, and in the presence of more than 1 and less than 25 mole percent based on the olefinic alcohol of the titanium chiral glycol catalyst.

The olefin of the olefinic alcohol is 0 carbon atoms from the carbinol carbon atom. The allylic alcohol can vary widely, being at least three carbon atoms and not more than about 60 carbon atoms, more usually not more than about 36 carbon atoms. However, polymers of allylic alcohols may be employed where some or a plurality of the olefinic groups may be epoxidized in accordance with the subject invention. The alcohol may be aliphatic, alicyclic, aralkyl or heterocyclic substituted alkyl or heterocyclic, where the heteroatoms do not interfere with the reaction, such as oxy ether, sulfone, polysubstituted amino, usually 3 to 4 substituents, or the like.

The allylic groups may be mono-, di-, tri- or tetra-substituted ethylenes with aliphatic, alicyclic or aromatic groups, particularly aliphatic and alicyclic groups, and the olefin may be exocyclic or endocyclic.

The carbinol carbon should be free of active functionalities such as basic amines and acidic groups.

In view of the enormous diversity of carbinols which may be employed as substrates, only various classes of products of interest can be suggested as useful in the subject process, either for synthesis of a particular product, for preparing an intermediate, or for modification of an existing product, particularly a natural product.

The subject invention can be used in organic synthesis of enantiomers, both for introduction of a variety of functionalities in a synthetic procedure and optical resolution of an enantiomer from a racemic mixture. The sole requirements for use of the subject invention and syntheses and/or resolution are the presence of (1) an hydroxyl group; (2) a chiral or prochiral center, which may or may not involve the carbinol carbon; and (3) an olefin.

General classes of compounds of interest include steroids, lipids, prostaglandins, terpenoids, hormones, saccharides, CBS drugs, $\alpha$- and $\beta$-adrenergic blocking agents, antiarrythmic drugs, vasodilator drugs, analgesics, antibiotics, and the like. Of particular interest are those compounds which have a three carbon unit, where the three carbon unit includes at least two oxy functionalities, at least one of which has a chiral carbon, and the third carbon may lack a heterofunctionality, or have a heterofunctionality, such as amino, thio, or the like.

The catalyst which is employed is extensively described in US-A-4,471,130. The subject invention uses a titanium chiral glycol alkoxide catalyst, that is, the catalyst will employ quadrivalent titanium ($Ti^{+4}$) ) chelated with an asymmetric glycol, where the glycol may be substituted with heteroatom containing functionalities or unsubstituted with heteroatom containing functionalities. The glycol will be at least 4 carbon atoms and may be up to about 36 carbon atoms, usually not more than about 30 carbon atoms, and preferably not more than about 20 carbon atoms.

Of particular interest as the glycol are derivatives of tartaric acid, both esters and amides, where the esters may have alkoxy groups of from 1 to 12, usually 1 to 6, more usually 1 to 3 carbon atoms, preferably methyl, ethyl and isopropyl, and the amides may have unsubstituted, mono- or disubstituted amino groups, where the substituents on the nitrogen may be from about 1 to 12 carbon atoms, usually from 1 to 6 carbon atoms, more usually from about 1 to 3 carbon atoms.

The glycol should be free of groups which interfere with the reaction, groups which react with the peroxide under the conditions of the epoxidation, groups which might displace the asymmetric glycol from the titanium, or the like.

In addition to the glycol, alkoxides are employed having a single oxy functionality linked to titanium, where the alkoxide will normally be non-chiral and free of functionalities which might interfere with the reaction. For the most part, the alkoxides will be aliphatic alcohols of from 1 to 12, generally of from 1 to 6, and preferably of from 3 to 6 carbon atoms. Preferably, the alkoxides will be relatively bulky, being branched at the $\alpha$ carbon atom, normally having from 1 to 2 branches. Particularly preferred is isopropoxide and tert.-butoxide. It should be clearly understood that the alkanols are primarily chosen for convenience and any alkanol may be employed, so long as the substituents on the alkanol do not interfere with the course of the reaction.

The preparation of the catalyst is conventional and can be performed in situ. The titanium tetraalkoxide may be combined with the chiral glycol in approximately stoichiometric amounts in an inert solvent. There is no requirement that the alcohol which is formed be removed, although it may be removed, for example, by distillation.

The hydroperoxides which are employed for the epoxidation may be aliphatic, alicyclic, or aralkyl hydroperoxides, preferably being tertiary hydroperoxides, more preferably having at least two aliphatic carbon atoms bonded to the carbon bonded to the hydroperoxide group. The hydroperoxide will generally be from about 3 to 20 carbon atoms, more usually from about 3 to 12 carbon atoms, particularly hydroperoxides having acceptable thermostability.

Illustrative hydroperoxides include t.-butyl hydroperoxide, $\alpha,\alpha$-dimethylheptyl hydroperoxide, bis-diisobutyl-2,5-dihydroperoxide, 1-methylcyclohexyl hydroperoxide, cumene hydroperoxide, cyclohexyl hydroperoxide, and trityl hydroperoxide.

As the reaction medium, inert solvents will be employed, particularly halohydrocarbon solvents of from 1 to 3 carbon atoms, preferably from 1 to 2 carbon atoms. A solvent which has been found to be quite satisfactory is methylene dichloride. All of the materials employed should be substantially free of water prior to being added to the reaction mixture. In addition, any water accumulation should be inhibited by the rapid removal of any water.

Water can be directly removed by adding to the reaction mixture activated molecular sieves which will strongly bind water, so as to substantially remove its activity as a hydrolyzing agent. Conveniently, zeolites may be employed as a powder or small particles which may be introduced in the mixture in sufficient amount to absorb any of the water which is formed. Zeolites of interest include zeolite 3A, zeolite 4A, and zeolite 5A.

Mild conditions will normally be employed, with temperatures below about 80°C, usually below about 30°C, and generally in the range of about -100° to 20°C, more usually in the range of about -50° to 10°C. The reaction is desirably carried out under an inert atmosphere, conveniently nitrogen.

In carrying out the epoxidation, the activated molecular sieve will be added prior to the occurrence of any significant amount of reaction. For best results the molecular sieve is introduced prior to or at the beginning of the catalyst preparation. It is found that rates of epoxidation are enhanced by preparation of the catalyst in the presence of the activated molecular sieve. The amount to be employed will vary and may be determined empirically. The amount may vary from 5 to 1000, usually from about 100 to 500 weight percent based on catalyst. All of the activated molecular sieve may be added initially or portions may be added from time to time.

The titanium catalyst can be pre-prepared or conveniently prepared in situ. In preparing the catalyst, the titanium alkoxide is combined with the chiral glycol in a dry inert solvent, preferably in the presence of the activated molecular sieve, at a temperature below about 30°C and in about stoichiometric proportions, although small excesses, generally less than about 100, usually less than about 50 mole percent excess, of the chiral glycol may be employed. The reaction is then allowed to proceed for a sufficient time for the catalyst to form. Particularly, titanium tetraisopropoxide may be combined with tartrate diester, diamide or half-ester amide.

Usually, the reaction forming the catalyst is complete in less than about 30min, more usually in less than about 15min. The time is not critical and can be optimized for a particular set of conditions and materials. The amount of catalyst employed will generally be less than about 25 mole percent based on olefinic alcohol, more usually less than about 20 percent and usually in excess of about 1 percent, generally from about 3 to 12 mole percent, more usually from about 5 to 10 mole percent.

The hydroperoxide is normally added in at least stoichiometric amount based on total olefinic alcohol, and preferably in excess, usually at least 25 percent excess, more usually at least 50 percent excess and not more than about 500 percent excess, that is, about 1 to 5 equivalents, preferably from about 1 to 2 equivalents of the hydroperoxide will be used per olefin equivalent. Of particular interest is the use of either tert.-butyl hydroperoxide or aralkyl t.-hydroperoxide, e.g., cumene hydroperoxide, the latter finding particular use with low molecular weight olefinic alcohols.

The concentrations of the various materials may be varied widely, the olefinic alcohol normally being from about 0.005 to about 2M, preferably being from about 0.05 to 1M. The concentrations of the other reactants will be related accordingly.

The hydroperoxide reacts with the catalyst in a reaction which is usually complete in less than about lhr, usually less than about 30min. The order of addition is not critical, so that the hydroperoxide and olefinic carbinol can be added consecutively in any order or concurrently. Where the hydroperoxide is added initially, the olefinic carbinol may be added after completion of the reaction between the catalyst and hydroperoxide.

After all the reactants have been combined in the inert medium in the presence of the dehydrating agent, the reaction is continued until the substrate has been transformed to the desired degree. The rate of the reaction varies depending upon the conditions and the amount of material involved and may vary from a few minutes to a few days. By employing continuous dehydration premature deactivation of the catalyst is prevented, rates are accelerated, so that shorter times are required than when carrying out the reaction in the absence of continual dehydration, even in the presence of higher amounts of catalyst. Reactions are frequently complete within about 2 to 3hr at -20°C.

After completion of the reaction, the reaction may be worked up in a variety of conventional ways. The catalyst may be destroyed using a mildly acidic or basic aqueous solution and where the epoxidized olefin is in the organic layer, the organic layer is isolated, dried, and the product isolated. For water soluble products, salting out, extraction, chromatography or distillation may be required. purification of the product may then be carried out in accordance with conventional means.

If desired, the epoxide may be further reacted to form the monoester. By employing an acylating agent, e.g., carboxylating, sulfonylating, etc., which has been or is activated, e.g., acyl halide or anhydride in the presence of a tertiary amine, at temperatures in the range of about -50° to 20°C, the monoester epoxide may be obtained. The product may then be isolated by conventional work up.

Alternatively, the epoxide ring may be opened by employing a combination of a nucleophile and at least a stoichiometric amount of a metal ester, e.g., titanate, zirconate, etc., particularly aliphatic esters, more particularly alkyl esters. The metal esters provide for improved regioselectivity. The reaction is carried out under mild conditions, generally from about -20° to 80°C, usually from about 0° to 40°C. The nucleophile will usually be in greater than stoichiometric amount, usually at least 1-fold excess. Nucleophiles include halide ions, amines, azide, mercaptans, mercaptides, selenides, cyanide, carboxylates, alkoxides, phenoxides, or the like. Various organic solvents may be employed, both polar and non-polar. Enhanced regioselectivity is achieved, with opening preferably occurring at the 3-carbon atom from the hydroxyl of the glycidyl alcohol. Prior to the epoxide opening, any hydroperoxide present may be destroyed, using various conventional reagents.

The following examples are offered by way of illustration and not by way of limitation.

## EXPERIMENTAL

### Example 1 - Exemplary Reactions, Reagents and Equipment

(1) All glass equipment was flame-dried under vacuum just prior to use, however this was found generally not to be necessary.

(2) Molecular sieves used in the reaction were of commercial grade, purchased as the activated (4A) or unactivated (3A) 2-3µ powder (Aldrich). Molecular sieves used for storage or drying of solvents were in the pellet form (Linde). Activation of the unactive molecular sieves was accomplished by heating at 250-300° for several hours under 0.05mm pressure.

(3) The dichloromethane used as reaction solvent was generally freshly distilled from calcium hydride prior to use, however this was observed to be necessary only for small scale (<10mmol) reactions.

(4) Dialkyl tartrate was distilled, stored under argon or nitrogen, and dispensed by gastight syringe.

(5) Titanium(IV)isopropoxide was of commercial grade and dispensed by gastight syringe.

(6) Tert.-butyl hydroperoxide (TBHP) was used as a 50% solution in dichloromethane, prepared from 70% aqueous TBHP by dilution with dichloromethane, phase separation, azeotropic removal of water, and sequential drying over molecular sieves. The solution was stored at -20° over 3A or 4A molecular sieve pellets. Just prior to use, somewhat more than the required amount of this solution was allowed to stand over a small amount of activated 3A or 4A molecular sieve pellets at room temperature for several minutes.

(7) All allylic alcohols were synthesized, except for geraniol, which was of commercial grade, and cinnamyl alcohol, which was distilled at 0.2mm. All alcohols were dissolved in dichloromethane (about three volumes) and dried as described above for TBHP just prior to use.

(8) Reactions were monitored by either thin layer chromatography (silica, petroleum ether/ethyl acetate:4/1 or petroleum ether/ethyl ether:1/1; anisaldehyde/sulphuric acid/ethanol developer) or capillary GPLC (SE-30, helium, 60-120°).

(9) The reactions were quenched in one of five ways:

(a) For analysis of enantiomeric purity, 100µL of the solution was withdrawn by syringe and quenched into a solution of 5mg of p-N,N-dimethylaminopyridine in 100µL of triethylamine. (-)-α-Methoxy-α-(trifluoromethyl)phenylacetyl chloride (20µL) or acetic anhydride (50µL) was then added, and the mixture was shaken for lmin. Chromatography (70x5mm silica, petroleum either at first, then petroleum ether/ethyl ether(9/1) to elute) provided material of sufficient purity for NMR analysis. Analysis was accomplished using 250MHz $^1$H NMR (C6D6). For the analysis of acetates, the shift reagent Eu(hfc)$_3$ was employed.

(b) Alternatively, for analysis of reaction extent, quenching into a mixture of 25µL of sat. aq. sodium sulfate and 100µL of ethyl ether, diluting with 400µL of ethyl ether, and filtration gave a solution which could be analyzed by capillary GC (22m SE-30, 60-140°, helium carrier).

(c) For purposes of isolation of the epoxy alcohol, the reaction was quenched by addition of ethyl ether and 10% sodium hydroxide saturated with brine as described below.

(d) For purposes of isolation of the epoxy ester, the reaction mixture was treated with 2-3 eq. of triethylamine, 0.02 eq. of p-N,N-dimethylaminopyridine, and 1-3 eq. of acylating reagent (e.g., acetic anhydride or tosyl chloride) and stirred at 0° or room temperature for 1 to 4hr. Evaporation of solvent and chromatography or distillation provided the pure ester.

(e) For purposes of isolation of a nucleophilically epoxide opened product, the reaction was quenched with 1-2 equivalents of a reducing agent (phosphine, phosphite, sulfide, hydride, etc.) then treated with the nucleophile (1-2 eq.) and titanium isopropoxide (1.2 eq.) as described below in Examples 4 and 5.

(10) Phosphate buffer (3M, pH7) was prepared by dissolving 1.5mol of disodium hydrogen phosphate and 1.5mol of potassium dihydrogen phosphate in enough water to make lL of solution, then adding sodium hydroxide to bring the pH to 7.0.

Table 1 indicates the results, employing the above-described procedure.

TABLE 1

| alcohol | %Ti | %tart | tartrate | run time | %yield | %ee |
|---|---|---|---|---|---|---|
| trans-2-hexen-1-ol | 5 | 6 | Et | 40 min | >95[b] | 90 |
| trans-2-hexen-1-ol | 10 | 12 | Et | 40 min | >95[b] | 93 |
| trans-2-hexen-1-ol | 5 | 7.5 | Et | 2 hr | 80[c] | 92 |
| trans-2-decen-1-ol | 5 | 7.5 | Et | 2 hr | 96 | >95 |
| cis-2-decen-1-ol | 10 | 15 | Et | 30 hr | 90 | 86 |
| cis-2-butene-1,4-diol monobenzyl ether | 10 | 12 | Et | 3 days | >95 | 85 |
| geraniol | 5 | 6 | Et | 15 min | >95 | 86 |
| geraniol | 5 | 7.5 | Et | 2 hr[d] | >95 | 93 |
| 1-hydroxymethylcyclohexene | 5 | 7.5 | Et | 3 hr[d] | 77[c] | 92 |
| allyl alcohol | 5 | 6 | iPr | 5 hr[e] | 98[b] | 89 |

[a] All yields are for isolated material of >95% purity by 250MHz nmr, except as noted.
[b] GC yield.
[c] Isolated yield; loss due to volatility.
[d] Reaction carried out at -40° after catalyst preparation at -20°.
[e] Reaction carried out at 0° after catalyst preparation at -5°.

Example 2 - (2S,3S)-3-heptyloxiranemethanol

A flame-dried 100mL round bottom 3-necked (RB3N) flask (note 1) fitted with thermometer, stopcock, septum, and stirbar was purged with argon, charged with 4A molecular sieves (300mg; note 2) and 30mL of dichloromethane (note 3), and cooled to -22° (30% ethylene glycol/water/dry ice). R,R-(+)-diethyl tartrate (97mg; 47mmol; note 4) and titanium (IV)isopropoxide (91mg; 0.32mmol; note 5) were added, and the mixture was stirred at -22±2° for 10min. t-Butyl hydroperoxide (2.2mL of a 5.8M dichloromethane solution; note 6) was added, and the mixture was stirred at -22±2° for 30min. A solution of trans-2-decen-1-ol (1.00g; 6.4mmol; note 7) in dichloromethane, was then added. The mixture was stirred under argon at -22±2° until analysis (note 8) indicated >95% reaction (2.5hr).

The reaction was quenched (note 9) with a 10% aqueous sodium hydroxide solution saturated with sodium chloride (0.75mL), diluted with 15mL of anhydrous ethyl ether, removed from the cold bath, and with vigorous stirring, allowed to warm to 10°. After filtration through diatomaceous earth, the clear filtrate was retreated with 0.75mL of 10% sodium hydroxide/brine solution, this time stirring at room temperature for 15min. Neutralization with 0.5mL of phosphate buffer (note 10), treatment with 1.5g of diatomaceous earth and 1.5g of anhydrous magnesium sulphate, filtration through diatomaceous earth, and evaporation of solvent gave a white powder (1.10g, 100%, mp 43.5-48.5°), which by 250MHz $^1$H nmr was of >95% chemical and optical purity. Recrystallization from petroleum ether provided white needles, mp 49.5-50.0°. $[\alpha]_D^{25}$ -36.5° (c 2.8, HCCl$_3$, >97% ee).

Example 3 - Preparation of (2S)-glycidol

An oven-dried 500mL round bottom flask fitted with septum and stirbar was charged with 3A powdered molecular sieves (3.5g) and dichloromethane (190mL). Under a nitrogen atmosphere, R,R-(+)-diisopropyl tartrate (1.25mL, 5.95mmol) and allyl alcohol (6.8mL, 0.1mol) were added and the solution was cooled to -5°. Titanium(IV)isopropoxide (1.5mL, 5.0mmol) was added, and the mixture was stirred at -5±2° for 10-30min. Commercial grade 80% cumene hydroperoxide (0.2mol, dried over 3A powdered sieves) was added slowly over a period of 30min. The mixture was stirred under nitrogen at -3±2° until analysis by GPLC (carbowax capillary, 70°) indicated >95% reaction (5h).

The reaction was quenched by adding citric acid (1g) in ether (100 to 200mL). The cooling bath was removed and the mixture was stirred for 20-30min. After filtration through a pad of diatomaceous earth, the filtrate was concentrated and distilled at reduced pressure to give glycidol (0.065mol 65%, containing a small amount of cumene).

Example 4 - In-situ opening of glycidol by benzenethiol

The asymmetric epoxidation was performed in exactly the same way as described above using 0.55mL of allyl alcohol (8mmol). After 5-6hr at -3±2°, the reaction mixture was cooled to -25±5° and trimethyl phosphite (1.4mL, 12mmol) was added slowly. After stirring at -25±5° for 30min the mixture was treated with benzenethiol (1.0mL, 9.7mmol) and titanium(IV)isopropoxide (3.0mL, 10mmol). The mixture was slowly warmed to room temperature and stirred for lhr.

The mixture was diluted with ether (20 to 40mL), and 10% aqueous sulfuric acid (25 to 50mL) was added. The mixture was stirred vigorously until two clear phases formed (1hr). Phase separation, extraction with ethyl acetate and chromatographic purification provided (S)-3-phenylthio-1,2-propanediol as a white solid (1.290g, 88%, mp 79-81°, 89% ee).

Example 5 - In-situ opening of glycidol by 1-naphthol; synthesis of (S)-propranolol

The asymmetric epoxidation was performed in exactly the same way as described above using 6.8mL of allyl alcohol (0.1mol). After 6hr at -3±2° the reaction mixture was cooled to -25±5° and trimethyl phosphite (16mL, 0.135mol) was added slowly. The mixture was warmed to room temperature, then poured into a solution prepared under nitrogen from 1-naphthol (14.5g, 0.10mol) and NaH (2.4g, 0,10mol) in 400mL of t.-butanol. Titanium(IV)isopropoxide (36mL, 0.12mol) was added and the mixture was stirred overnight at room temperature under nitrogen.

The reaction mixture was filtered through a pad of diatomaceous earth. The filtrate was concentrated to 150mL and treated with 200mL of 10% aqueous sulfuric acid as described above. Phase separation, extraction, and concentration gave a crude oil, which was dissolved in 150mL of ether and treated with 100mL of 1N aqueous sodium hydroxide (45min at room temperature). Phase separation, extraction, and concentration provided crude (S)-3-(1-naphthoxy)-1,2-propanediol, which was carried on by known methods (Iriuchijima and Kojima, Agric. Biol. Chem. (1982) 46:1153) to give crude (S)-propranolol hydrochloride (21.63g, 73.2% from allyl alcohol). Recrystallization from methanol-ether afforded 15.00g of the product (50.8%). Further recrystallization from methanol-ether provided white crystals of enantiomerically pure (S)-propranolol hydrochloride (12.56g, mp 192.5-193.5°; $[\alpha]_D^{21}$ -25.7°, c 1.18, ethanol).

It is evident from the above results that the subject method provides a substantial improvement over the prior discovery as described in US-A-4,471,130 (EP-A-0046033). The rate of the reaction is enhanced, yields and enantiomeric efficiencies are improved, and greatly improved results are obtained with the small allylic alcohols which give water soluble epoxy alcohols. Furthermore, the isolation procedures are greatly simplified due to the much smaller amounts of tartrate ester and titanium containing compounds which must be removed at the end of the reaction. Thus, by using molecular sieves, which are inexpensive reagents, great economies are achieved in the epoxidation of a wide variety of alcohols. In this manner, the utility of the patented epoxidation method of US-A-4,471,130 is greatly expanded, so as to be applied to a wide variety of syntheses which would not otherwise be economical.

## Claims

1. A method for preparing epoxides, the method employing a titanium chiral glycol alkoxide catalyst, an allylic alcohol of which the olefin is separated from the carbinol carbon atom by 0 carbon atom, and an organic hydroperoxide in an inert dry medium whereby the olefin is asymmetrically epoxidized, characterised by:

   the presence of activated molecular sieves in the reaction mixture
   and by the presence of more than 1 and less than 25 mole % based on the allylic alcohol, of the titanium chiral glycol alkoxide catalyst.

2. A method according to claim 1 which is carried out under mild conditions.

3. A method according to claim 1 or claim 2, wherein said catalyst is prepared in the presence of the activated molecular sieves.

4. A method according to claim 1, claim 2 or claim 3, wherein the said hydroperoxide is tert.-butyl hydroperoxide.

**5.** A method according to claim 1, claim 2 or claim 3, wherein the said hydroperoxide is cumene hydroperoxide.

**6.** A method according to any one of the preceding claims, including the additional step of combining <u>in situ</u> the product of said epoxidizing with an acylated agent to produce the ester.

**7.** A method for preparing glycidol from allyl alcohol, wherein a titanium chiral glycol alkoxide catalyst is combined with allyl alcohol and an organic hydroperoxide in an inert anhydrous halohydrocarbon medium under mild conditions, whereby said allyl alcohol is epoxidized to glycidol, characterized by:

preparing said catalyst in the presence of activated molecular sieves; and
including activated molecular sieves in said inert medium
and by having said catalyst present in the amount of more than 1 and less than 25 mol % based on the allyl alcohol.

**8.** A method according to claim 7, wherein said hydroperoxide is cumene hydroperoxide.

**9.** A method according to any one of claims 1 to 6 wherein the epoxide is a water-soluble epoxy alcohol.

**Patentansprüche**

**1.** Verfahren zur Herstellung von Epoxiden, wobei das Verfahren ein Titanalkoxid eines chiralen Glykols als Katalysator, einen Allylalkohol, dessen Olefin vom Carbinolkohlenstoff um 0 Kohlenstoffatome entfernt ist, und ein organisches Hydroperoxid in einem trockenen Inertmedium verwendet, wodurch das Olefin asymmetrisch epoxidiert wird, gekennzeichnet durch:

die Gegenwart aktivierter Molekularsiebe im Reaktionsgemisch

und durch die Gegenwart von mehr als 1 und weniger als 25 Mol-% (bezogen auf den Allylalkohol) des Titanalkoxids eines chiralen Glykols als Katalysator.

**2.** Verfahren nach Anspruch 1, das unter milden Bedingungen durchgeführt wird.

**3.** Verfahren nach Anspruch 1 oder 2, worin der Katalysator in der Gegenwart der aktivierten Molekularsiebe hergestellt wird.

**4.** Verfahren nach Anspruch 1, 2 oder 3, worin das Hydroperoxid tert-Butylhydroperoxid ist.

**5.** Verfahren nach Anspruch 1, 2 oder 3, worin das Hydroperoxid Cumolhydroperoxid ist.

**6.** Verfahren nach einem der vorhergehenden Ansprüche, umfassend den zusätzlichen Schritt des in situ-Kombinierens des Produkts der Epoxidierung mit einem acylierten Agens, um den Ester herzustellen.

**7.** Verfahren zur Herstellung von Glycidol aus Allylalkohol, worin ein Titanalkoxid eines chiralen Glykols als Katalysator mit Allylalkohol und einem organischen Hydroperoxid in einem wasserfreien Halogenkohlenwasserstoff-Inertmedium unter milden Bedingungen kombiniert wird, wodurch der Allylalkohol zu Glycidol epoxidiert wird, gekennzeichnet durch:

die Herstellung des Katalysators in der Gegenwart aktivierter Molekularsiebe; und

das Vorhandensein aktivierter Molekularsiebe im Inertmedium sowie

das Bereitstellen des Katalysators in einer Menge von mehr als 1 und weniger als 25 Mol-% (bezogen auf den Allylalkohol).

**8.** Verfahren nach Anspruch 7, worin das Hydroxyperoxid Cumolhydroxyperoxid ist.

**9.** Verfahren nach einem der Ansprüche 1 bis 6, worin das Epoxid ein wasserlöslicher Epoxyalkohol ist.

## Revendications

1. Méthode de préparation d'époxydes, la méthode employant un catalyseur alcoxyde glycol chiral de titane, un alcool allylique dont l'oléfine est séparée de l'atome de carbone carbinol par zéro atome de carbone, et un hydroperoxyde organique dans un milieu sec inerte ce par quoi l'oléfine est époxydée asymétriquement, caractérisée par :

   la présence de tamis moléculaires activés dans le mélange réactionnel
   et par la présence de plus de 1 et moins de 25% en moles sur la base de l'alcool allylique, du catalyseur alcoxyde glycol chiral de titane.

2. Méthode selon la revendication 1 qui est mis en oeuvre dans des conditions douces.

3. Méthode selon la revendication 1 ou la revendication 2, dans laquelle ledit catalyseur est préparé en présence des tamis moléculaires activés.

4. Méthode selon la revendication 1, la revendication 2 ou la revendication 3, dans laquelle ledit hydroperoxyde est du tert-butyl hydroperoxyde.

5. Méthode selon la revendication 1, la revendication 2 ou la revendication 3, dans laquelle ledit hydroperoxyde est l'hydroperoxyde de cumène.

6. Méthode selon l'une quelconque des revendications précédentes, incluant l'étape additionnelle de combinaison in situ du produit de ladite époxydation avec un agent acylaté pour produire l'ester.

7. Méthode pour préparer un glycidol à partir d'un alcool allylique, dans laquelle un catalyseur alcoxyde glycol chiral de titane est combiné avec l'alcool allylique et un hydroperoxyde organique dans un milieu halohydrocarbure anhydre inerte dans des conditions douces, ce par quoi ledit alcool allylique est époxydé en glycidol, caractérisée par :

   la préparation dudit catalyseur en présence de tamis moléculaires activés; et
   l'inclusion des tamis moléculaires activés dans le milieu inerte
   et en ce que on a ledit catalyseur présent en la quantité de plus de 1 et moins de 25% en moles sur la base de l'alcool allylique.

8. Méthode selon la revendication 7, dans laquelle ledit hydroperoxyde est de l'hydroperoxyde de cumène.

9. Méthode selon l'une quelconque des revendications 1 à 6, dans laquelle l'époxyde est un alcool époxy soluble dans l'eau.